(19) Europäisches Patentamt
European Patent Office
Office européen des brevets

(11) EP 0 612 759 B1

(12) EUROPÄISCHE PATENTSCHRIFT

(45) Veröffentlichungstag und Bekanntmachung des Hinweises auf die Patenterteilung:
**02.10.1996 Patentblatt 1996/40**

(51) Int Cl.[6]: **C07H 23/00**, C08G 77/38, B01F 17/00

(21) Anmeldenummer: **94102709.6**

(22) Anmeldetag: **23.02.1994**

(54) **Glycosidreste aufweisende Organosiliciumverbindungen und Verfahren zu deren Herstellung**

Glycoside containing organosilicon compounds, and method for their preparation

Composés organosilicié comportant un reste glycosidique et procédé pour leur préparation

(84) Benannte Vertragsstaaten:
**AT BE CH DE ES FR GB IT LI NL**

(30) Priorität: **26.02.1993 DE 4306041**

(43) Veröffentlichungstag der Anmeldung:
**31.08.1994 Patentblatt 1994/35**

(73) Patentinhaber: **Wacker-Chemie GmbH**
**D-81737 München (DE)**

(72) Erfinder:
- **Sejpka, Johann, Dr.**
  **D-84533 Marktl (DE)**
- **Wimmer, Franz**
  **D-84489 Burghausen (DE)**

(56) Entgegenhaltungen:
**EP-A- 0 416 402**
**AT-B- 393 509**
**US-A- 3 839 318**

EP 0 612 759 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft Glycosidreste aufweisende Organosiliciumverbindungen, Verfahren zu deren Herstellung sowie deren Verwendung.

Alkyl(poly)glycoside und Alkylpolyalkoxyalkylglucoside und deren Verwendung als Tenside, Emulgatoren und Schaumstabilisatoren sind bereits bekannt. Hierzu sei beispielsweise auf US 3,219,656 A (Rohm & Haas Co.; ausgegeben am 23. November 1965), US 4,950,743 A (Henkel KGaA; 21. August 1990) und DE 39 25 846 A (Hüls AG; ausgegeben am 14. Februar 1991) bzw. die entsprechende US 5,133,897 (ausgegeben am 28. Juli 1992) verwiesen.

Die AT 393 509 beschreibt hydrophile Silane bzw. lineare und vernetzte Polysiloxane, die durch Hydrosilylierung von ungesättigten Kohlenhydrat-Derivaten mit H-Si-funktionellen Silanen bzw. Polysiloxanen hergestellt werden.

Gegenstand der Erfindung sind Glycosidreste aufweisende Organosiliciumverbindungen aus Einheiten der Formel

$$R^1{}_xR_{3-x}SiO\text{-}[(SiRR^1O)_m\text{-}(SiR_2O)_n]_y\text{-}SiR_{3-x}R^1{}_x \qquad (III),$$

R gleich oder verschieden sein kann und Wasserstoffatom oder organischer Rest bedeutet,
$R^1$ gleich oder verschieden sein kann und einen Rest der Formel

$$Z\text{-}(R^2O)_c\text{-}R^3\text{-} \qquad (II)$$

bedeutet, worin
Z einen Glycosidrest bedeutet, der aus 1 bis 10, vorzugsweise 1 bis 4, besonders bevorzugt 1 bis 2, Monosaccharideinheiten aufgebaut ist,
$R^2$ gleich oder verschieden sein kann und Alkylenrest bedeutet, c 0 oder eine Zahl von 1 bis 20, bevorzugt 0 oder eine Zahl von 1 bis 15, besonders bevorzugt 0 oder eine Zahl von 1 bis 4, ist und
$R^3$ Alkylenrest bedeutet,
m gleich oder verschieden sein kann und 0 oder eine Zahl von 1 bis 200, bevorzugt 0 oder eine Zahl von 1 bis 100, besonders bevorzugt 0 oder eine Zahl von 1 bis 50, ist, Zahl von 1 n gleich oder verschieden sein kann und o oder eine Zahl von 1 bis 1000, bevorzugt 0 oder eine Zahl von 1 bis 500, besonders bevorzugt 0 oder eine Zahl von 1 bis 100, ist,
x 0 oder 1 ist und
y 0 oder eine Zahl von 1 bis 1200, bevorzugt 0 oder eine Zahl von 1 bis 600, besonders bevorzugt 0 oder eine Zahl von 1 bis 100, ist,

mit der Maßgabe, daß die Verbindung der Formel (III) mindestens einen Rest $R^1$ aufweist. Bevorzugt handelt es sich bei Rest R um gegebenenfalls substituierte Kohlenwasserstoffreste mit 1 bis 18 Kohlenstoffatomen, wobei Alkylreste mit 1 bis 4 Kohlenstoffatomen, insbesondere der Methylrest, besonders bevorzugt sind.

Beispiele für Reste R sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, Octadecylreste, wie der n-Octadecylrest; Alkenylreste, wie der Vinyl-, Allyl-, n-5-Hexenyl-, 4-Vinylcyclohexyl- und der 3-Norbornenylrest; Cycloalkylreste, wie Cyclopentyl-, Cyclohexyl-, 4-Ethylcyclohexyl-, Cycloheptylreste, Norbornylreste und Methylcyclohexylreste; Arylreste, wie der Phenyl-, Biphenylyl-, Naphthyl- und Anthryl- und Phenanthrylrest; Alkarylreste, wie o-, m-, p-Tolylreste, Xylylreste und Ethylphenylreste; Aralkylreste, wie der Benzylrest, der $\alpha$- und der $\beta$-Phenylethylrest.

Beispiele für Monosaccharide, aus denen die Glycosidreste Z aufgebaut sein können, sind Hexosen und Pentosen, wie Glucose, Fructose, Galactose, Mannose, Talose, Allose, Altrose, Idose, Arabinose, Xylose, Lyxose und Ribose, wobei Glucose besonders bevorzugt ist.

Beispiele für Alkylenreste sind Methylen-, Ethylen-, Propylen-, Butylen-, Pentylen-, Hexylen-, Heptylen-, Octylen-, Nonylen-, Decylen- und Octadecylenreste.

Bevorzugt handelt es sich bei Rest $R^2$ um den Ethylenrest und den 1,2-Propylenrest, wobei der Ethylenrest besonders bevorzugt ist.

Bevorzugt handelt es sich bei Rest $R^3$ um lineare Alkylenreste mit 2 bis 20 Kohlenstoffatomen, besonders bevorzugt um lineare Alkylenreste mit 2 bis 8 Kohlenstoffatomen, insbesondere um den n-Propylenrest.

Beispiele für Reste $R^1$ sind

$$G\text{-}CH_2CH_2CH_2\text{-},$$

$$G\text{-}(CH_2CH_2O)\text{-}CH_2CH_2CH_2\text{-},$$

$$G\text{-}(CH_2CH_2O)_2\text{-}CH_2CH_2CH_2\text{-},$$

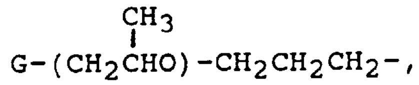

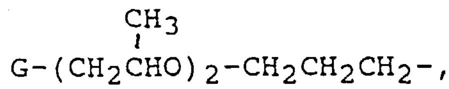

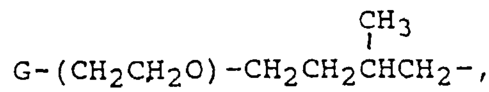

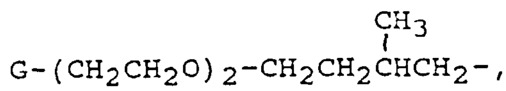

wobei G einen Glucosidrest ($C_6H_{11}O_6$-) bedeutet,

$$G_2\text{-}CH_2CH_2CH_2\text{-},$$

$$G_2\text{-}(CH_2CH_2O)\text{-}CH_2CH_2CH_2\text{-},$$

$$G_2\text{-}(CH_2CH_2O)_2\text{-}CH_2CH_2CH_2\text{-},$$

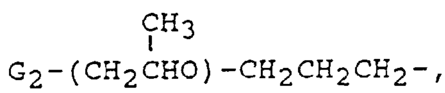

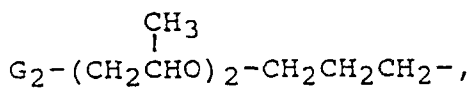

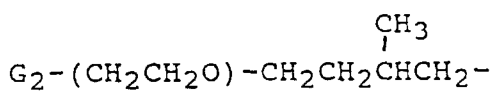

und

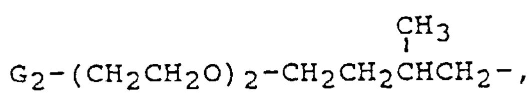

wobei $G_2$ einen aus zwei Glucoseeinheiten autgebauten Glycosidrest bedeutet.

Bevorzugt handelt es sich bei Rest $R^1$ um $G\text{-}CH_2CH_2CH_2$-, $G\text{-}(CH_2CH_2O)\text{-}CH_2CH_2CH_2$-, $G_2\text{-}CH_2CH_2CH_2$- und $G_2\text{-}(CH_2CH_2O)\text{-}CH_2CH_2CH_2$-, wobei $G\text{-}(CH_2CH_2O)\text{-}CH_2CH_2CH_2$-, und $G_2\text{-}(CH_2CH_2O)\text{-}CH_2CH_2CH_2$- besonders bevorzugt sind und G einen Glucosidrest ($C_6H_{11}O_6$-) und $G_2$ einen aus zwei Glucoseeinheiten aufgebauten Glycosidrest bedeutet.

Ist in den Glycosidresten aufweisenden Organosiliciumverbindungen gemäß Formel (III) m durchschnittlich verschieden 0, bedeutet x bevorzugt 0.

Ist in den Glycosidresten aufweisenden Organosiliciumverbindungen gemäß Formel (III) x durchschnittlich verschieden 0, ist m bevorzugt 0.

Obwohl durch Formel (III) nicht dargestellt, können bis zu 10 Molprozent der Diorganosiloxaneinheiten durch andere Siloxaneinheiten, wie beispielsweise $RSiO_{3/2}$-, $R^1SiO_{3/2}$-und/oder $SiQ_{4/2}$-Einheiten, ersetzt sein, wobei R und $R^1$ die oben dafür angegebene Bedeutung haben.

Die erfindungsgemäßen, Glycosidreste aufweisenden Organosiliciumverbindungen können nach verschiedenen Verfahren hergestellt werden.

**Verfahren 1**

Ein weiterer Gegenstand der Erfindung ist ein Verfahren zur Herstellung der erfindungsgemäßen, Glycosidreste aufweisenden Organosiliciumverbindungen, dadurch gekennzeichnet, daß in einer

1. Stufe

Mono- und/oder Oligosaccharid (1) mit Verbindung (2) der Formel

$$HO\text{-}(R^2O)_c\text{-}R^4 \qquad (IV),$$

worin

$R^2$ gleich oder verschieden sein kann und Alkylenrest bedeutet,
c 0 oder eine Zahl von 1 bis 20, bevorzugt 0 oder eine Zahl von 1 bis 15, besonders bevorzugt 0 oder eine Zahl von 1 bis 4, ist und
$R^4$ einen Alkenylrest bedeutet,

umgesetzt wird und in einer

2. Stufe

das in der 1. Stufe erhaltene Glycosid mit Si-gebundenen Wasserstoff aufweisender Organosiliciumverbindung (3) aus Einheiten der Formel

$$R^5_dH_eSiO_{\frac{4-d-e}{2}} \qquad (V)$$

umgesetzt wird, worin

$R^5$ gleich oder verschieden sein kann und organischer Rest bedeutet,
d 0, 1, 2 oder 3 ist und
e 0, 1, 2 oder 3 ist,

mit der Maßgabe, daß die Summe aus d und e kleiner oder gleich 3 ist und Organosiliciumverbindung aus Einheiten der Formel (V) pro Molekül mindestens ein Si-gebundenes Wasserstoffatom enthält.

Beispiele für die Saccharide (1) sind die oben für Monosaccharide genannten Beispiele, Sucrose, Lactose, Maltose und Raffinose sowie deren Hydrate, wobei in der 1. Stufe des erfindungsgemäßen Verfahrens 1 besonders bevorzugt Glucose bzw. Glucose Monohydrat eingesetzt wird.

Bevorzugt handelt es sich bei Rest $R^4$ um ω-Alkenylgruppen, wie den Vinyl-, Allyl-, 3-Butenyl- und den 3-Methyl-3-butenylrest, wobei der Allylrest besonders bevorzugt ist.

Beispiele für Verbindungen (2) sind

$HO\text{-}CH_2CH{=}CH_2$,

$HO\text{-}CH_2CH_2C(CH_3){=}CH_2$,

$HO\text{-}(CH_2CH_2O)\text{-}CH_2CH{=}CH_2$,

$HO\text{-}(CH_2CH_2O)_2\text{-}CH_2CH{=}CH_2$,

$HO\text{-}(CH_2CH(CH_3)O)\text{-}CH_2CH{=}CH_2$,

$HO\text{-}(CH_2CH(CH_3)O)_2\text{-}CH_2CH{=}CH_2$,

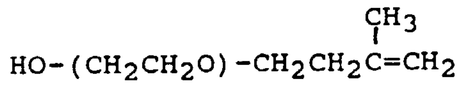

und

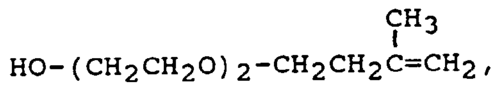

wobei

$HO-CH_2CH=CH_2$,

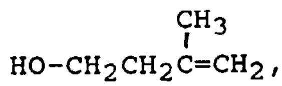

$HO-(CH_2CH_2O)-CH_2CH=CH_2$

und $HO-(CH_2CH_2O)_2-CH_2CH=CH_2$ bevorzugt sowie $HO-CH_2CH=CH_2$ und $HO-(CH_2CH_2O)-CH_2CH=CH_2$ besonders bevorzugt sind.

Bevorzugt handelt es sich bei der in der 2. Stufe des erfindungsgemäßen Verfahrens 1 eingesetzten Organosiliciumverbindung (3) um α-Hydrogenorganopolysiloxane und α,ω-Dihydrogenorganopolysiloxane, wie beispielsweise

$H-Si(CH_3)_2-O-Si(CH_3)_3$,
$H-Si(CH_3)_2-O-Si(CH_3)_2-H$,
$H-[Si(CH_3)_2-O]_4-Si(CH_3)_2-H$,
$H-[Si(CH_3)_2-O]_9-Si(CH_3)_2-H$,
$H-Si(CH_3)_2-O-[Si(CH_3)_2-O]_{15}-Si(CH_3)_2-H$,
$H-Si(CH_3)_2-O-[Si(CH_3)_2-O]_{18}-Si(CH_3)_2-H$ und
$H-Si(CH_3)_2-O-[Si(C_2H_5)_2-O]_{10}-Si(CH_3)_2-H$,

sowie
α,ω-Triorganylsiloxygruppen und Si-gebundenen Wasserstoff aufweisende Organopolysiloxane, wie beispielsweise

$(CH_3)_3SiO-SiHCH_3O-Si(CH_3)_3$,
$(CH_3)_3SiO[SiHCH_3O]_2Si(CH_3)_3$,
$(CH_3)_3SiO[SiHCH_3O]_3Si(CH_3)_3$,
$(CH_3)_3SiO[SiHCH_3O]_4Si(CH_3)_3$ und
$(CH_3)_3SiO[Si(CH_3)_2O]_{50}[SiHCH_3O]_5Si(CH_3)_3$,

wobei $H-Si(CH_3)_2-O-Si(CH_3)_3$ und $(CH_3)_3SiO-SiHCH_3O-Si(CH_3)_3$ besonders bevorzugt sind.

Beispiele für Reste $R^5$ sind die für R oben angegebenen Beispiele.

Bei der 1. Stufe des erfindungsgemäßen Verfahrens 1 beträgt das molare Verhältnis von eingesetztem Saccharid (1) zu Verbindung (2) vorzugsweise 1:1 bis 1:4, besonders bevorzugt 1:2 bis 1:3.

Die 1. Stufe des erfindungsgemäßen Verfahrens 1 wird vorzugsweise in Anwesenheit von organischer oder anorganischer Säure durchgeführt.

Beispiele für derartige Säuren sind anorganische Säuren, wie HCl, $HClO_4$, $H_2SO_4$ und $H_3PO_4$, und organische Säuren, wie Essigsäure, Ameisensäure, Propionsäure, p-Toluolsulfonsäure, Methansulfonsäure, Trifluormethansulfonsäure, Dodecylbenzolsulfonsäure, wobei p-Toluolsulfonsäure und Trifluormethansulfonsäure besonders bevorzugt eingesetzt werden.

Bei dem erfindungsgemäßen Verfahren 1 wird Säure vorzugsweise in Mengen von 0,05 bis 5,0 Gew.-%, besonders bevorzugt 0,5 bis 2,0 Gew.-%, bezogen auf das Gesamtgewicht an Saccharid (1), eingesetzt.

Falls erwünscht, kann die Säure im Gemisch mit Wasser und/oder einem organischen Lösungsmittel zugegeben werden.

Die 1. Stufe des erfindungsgemäßen Verfahrens 1 wird bei einer Temperatur von vorzugsweise 85 bis 120°C, besonders bevorzugt 95 bis 110°C, und einem Druck von vorzugsweise 20 bis 150 hPa, besonders bevorzugt von 50 bis 120 hPa, durchgeführt.

Nach Beendigung der Umsetzung der 1. Stufe wird vorteilhafterweise die Säure neutralisiert und überschüssige

Verbindung (2) auf bekannte Art und Weise, beispielsweise durch Destillation, entfernt, wobei, falls erwünscht, vor der Destillation von überschüssiger Verbindung (2) die Reaktionsmischung mit organischem Lösungsmittel, welches höher siedet als Verbindung (2), versetzt werden kann.

Beispiele für Basen, die zur Neutralisation der Säure in der 1. Stufe des erfindungsgemäßen Verfahrens 1 eingesetzt werden können, sind Alkalihydroxide, wie Natriumhydroxid und Kaliumhydroxid, Alkalisiliconate, Amine, wie beispielsweise Methylamin, Dimethylamin, Ethylamin, Diethylamin, Triethylamin und n-Butylamin, Ammoniumverbindungen, wie etwa Tetramethylammoniumhydroxid, wobei Natriumhydroxid besonders bevorzugt ist.

Falls erwünscht, kann die Base im Gemisch mit Wasser und/oder einem organischen Lösungsmittel zugegeben werden.

Die in der 1. Stufe des erfindungsgemäßen Verfahrens 1 erhaltenen Alkenylglycoside werden dann in einer 2. Stufe mit Organosiliciumverbindungen (3) in für die Anlagerung von Sigebundenen Wasserstoff an aliphatische Kohlenstoff-Kohlenstoff-Mehrfachbindung bekannten Art und Weise umgesetzt.

Bei der 2. Stufe des erfindungsgemäßen Verfahrens 1 liegt das Molverhältnis von Alkenylglycosid zu Organosiliciumverbindung (3) vorzugsweise bei 1 : 1 bis 1 : 2, besonders bevorzugt bei 1 : 1 bis 1 : 1,5.

Die Umsetzung der 2. Stufe wird vorzugsweise in Anwesenheit von die Anlagerung von Si-gebundenem Wasserstoff an aliphatische Mehrfachbindung fördernden Katalysator durchgeführt.

Als die Anlagerung von Si-gebundenem Wasserstoff an aliphatische Doppelbindung fördernde Katalysatoren können die gleichen Katalysatoren eingesetzt werden, die auch bisher zur Förderung der Anlagerung von Si-gebundenem Wasserstoff an aliphatische Doppelbindung eingesetzt werden konnten. Bei den Katalysatoren handelt es sich vorzugsweise um ein Metall aus der Gruppe der Platinmetalle oder um eine Verbindung oder einen Komplex aus der Gruppe der Platinmetalle.

Beispiele für solche Katalysatoren sind metallisches und feinverteiltes Platin, das sich auf Trägern, wie Siliciumdioxid, Aluminiumoxid oder Aktivkohle befinden kann, Verbindungen oder Komplexe von Platin, wie Platinhalogenide, z.B. $PtCl_4$, $H_2PtCl_6 \cdot H_2O$, $Na_2PtCl_4 \cdot 4H_2O$, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus $H_2PtCl_6 \cdot 6H_2O$ und Cyclohexanon, Platin-Vinylsiloxankomplexe, wie Platin1,3-Divinyl-1,1,3,3-tetramethyldisiloxankomplexe mit oder ohne Gehalt an nachweisbarem anorganisch gebundenem Halogen, Bis-($\gamma$-picolin)-platindichlorid, Trimethylendipyridinplatindichlorid, Dicyclopentadienplatindichlorid, Dimethylsulfoxydethylenplatin-(II)-dichlorid, Cyclooctadien-Platindichlorid, Norbornadien-Platindichlorid, $\gamma$-picolin-Platindichlorid, Cyclopentadien-Platindichlorid sowie Umsetzungsprodukte von Platintetrachlorid mit Olefin und primärem Amin oder sekundärem Amin oder primärem und sekundärem Amin, wie das Umsetzungsprodukt aus in 1-Octen gelöstem Platintetrachlorid mit sec.-Butylamin, wobei $PtCl_4$, $H_2PtCl_6 \cdot 6H_2O$ und Platin-Olefin-Komplexe bevorzugt und $H_2PtCl_6 \cdot 6H_2O$ besonders bevorzugt eingesetzt wird.

Der Katalysator wird vorzugsweise in Mengen von 0,1 bis 1000 Gew.-ppm, bevorzugt in Mengen von 1 bis 50 Gew.-ppm (Gewichtsteilen je Million Gewichtsteilen) eingesetzt, jeweils berechnet als elementares Platin und bezogen auf das Gesamtgewicht an Alkenylglycosid und Organosiliciumverbindung (3).

Die Umsetzung der 2. Stufe des erfindungsgemäßen Verfahrens 1 wird vorzugsweise bei einem Druck zwischen 900 und 1100 hPa und bei einer Temperatur von bevorzugt 50 bis 150°C, besonders bevorzugt 80 bis 140°C, durchgeführt.

Die 2. Stufe des erfindungsgemäßen Verfahrens 1 kann in Anwesenheit oder Abwesenheit von organischen Lösungsmitteln durchgeführt werden, wobei es sich vorzugsweise um solches handelt, in dem sich das in der 1. Stufe hergestellte Alkenylglycosid mindestens zu einem Teil, bevorzugt ganz, löst.

Beispiele für gegebenenfalls eingesetztes Lösungsmittel sind Toluol, Xylol und Isopropanol, wobei Toluol und Isopropanol besonders bevorzugt sind.

Bei dem erfindungsgemäßen Verfahren 1 wird vorzugsweise kein Lösungsmittel eingesetzt.

Nach einer bevorzugten Ausführungsform des erfindungsgemäßen Verfahrens 1 wird

in einer 1. Stufe ein Gemisch aus Glucose Monohydrat und Verbindung (2), insbesondere $HOCH_2CH_2OCH_2CH{=}CH_2$, unter vermindertem Druck auf etwa 100°C erhitzt, mit Säure, insbesondere p-Toluolsulfonsäure, versetzt und reagieren gelassen, wobei flüchtige Bestandteile destillativ entfernt werden, sowie

in einer 2. Stufe das in der 1. Stufe erhaltene Alkenylglycosid mit Organosiliciumverbindung (3) in Anwesenheit von Katalysator umgesetzt.

Das erfindungsgemäße Verfahren 1 hat den Vorteil, daß es einfach in der Durchführung ist und sehr hohe Ausbeuten erzielt werden. Des weiteren hat Verfahren 1 den Vorteil, daß gegebenenfalls eingesetztes Lösungsmittel sowie flüchtige Bestandteile durch die destillative Aufarbeitung rückgewonnen und wieder eingesetzt werden können.

**Verfahren 2**

Eine andere Möglichkeit zur Herstellung der erfindungsgemäßen, Glycosidreste aufweisenden Organosiliciumverbindungen besteht darin, daß zuerst Verbindung (2) mit Organosiliciumverbindung (3) umgesetzt und anschließend die so erhaltene hydroxyalkylfunktionelle Organosiliciumverbindung mit Saccharid (1) umgesetzt wird, was aber nicht bevorzugt ist.

Ein weiterer Gegenstand der Erfindung ist ein Verfahren zur Herstellung der erfindungsgemäßen, Glycosidreste aufweisenden Organosiliciumverbindungen, dadurch gekennzeichnet, daß in einer

1. Stufe

Verbindung (2) mit Organosiliciumverbindung (3) umgesetzt wird und dann in einer

2. Stufe

die in der 1. Stufe erhaltene Organosiliciumverbindung mit Saccharid (1) umgesetzt wird.

Beispiele, bevorzugte und besonders bevorzugte Spezies des Saccharids (1), der Verbindung (2) und der Organosiliciumverbindung (3) sind die für Verfahren 1 angegebenen Beispiele, bevorzugte und besonders bevorzugte Spezies.

Die 1. Stufe des erfindungsgemäßen Verfahrens 2 wird vorzugsweise in Anwesenheit von die Anlagerung von Si-gebundenem Wasserstoff an aliphatische Kohlenstoff-Kohlenstoff-Mehrfachbindung fördernden Katalysator durchgeführt. Beispiele, bevorzugte und besonders bevorzugte Spezies des Katalysators sind die für Verfahren 1 angegebenen Beispiele, bevorzugte und besonders bevorzugte Spezies.

Der Katalysator wird vorzugsweise in Mengen von 0,1 bis 1000 Gew.-ppm, bevorzugt in Mengen von 1 bis 50 Gew.-ppm eingesetzt, jeweils berechnet als elementares Platin und bezogen auf das Gesamtgewicht an Verbindung (2) und Organosiliciumverbindung (3).

Bei der 1. Stufe des erfindungsgemäßen Verfahrens 2 beträgt das Molverhältnis von Verbindung (2) zu Organosiliciumverbindung (3) vorzugsweise von 1 : 1 bis 1 : 2, besonders bevorzugt von 1 : 1 bis 1 : 1,5.

Die Umsetzung der 1. Stufe des erfindungsgemäßen Verfahrens 2 wird vorzugsweise bei einem Druck zwischen 900 und 1100 hPa und bei einer Temperatur von bevorzugt 50 bis 150°C, besonders bevorzugt 80 bis 140°C, durchgeführt.

Die 1. Stufe des erfindungsgemäßen Verfahrens 2 kann in Anwesenheit oder Abwesenheit von organischen Lösungsmitteln durchgeführt werden, wobei es sich um die oben für Verfahren 1 genannten organischen Lösungsmittel handeln kann. Vorzugsweise wird bei der 1. Stufe des Verfahrens 2 kein organisches Lösungsmittel eingesetzt.

Nach Beendigung der Umsetzung der 1. Stufe wird vorteilhafterweise überschüssige Verbindung (2) auf bekannte Art und Weise, beispielsweise durch Destillation, entfernt, wobei, falls erwünscht, vor der Destillation von überschüssiger Verbindung (2) die Reaktionsmischung mit organischem Lösungsmittel, welches höher siedet als Verbindung (2), versetzt werden kann.

Die in der 1. Stufe des erfindungsgemäßen Verfahrens 2 erhaltenen Organosiliciumverbindungen werden dann in einer 2. Stufe mit Saccharid (1) umgesetzt.

Bei der 2. Stufe des erfindungsgemäßen Verfahrens 2 beträgt das molare Verhältnis von eingesetztem Saccharid (1) zu der in der 1 Stufe hergestellten Organosiliciumverbindung vorzugsweise 1 : 1 bis 1 : 3, besonders bevorzugt 1 : 1 bis 1 : 2.

Die 2. Stufe des erfindungsgemäßen Verfahrens 2 wird vorzugsweise in Anwesenheit von organischer oder anorganischer Säure durchgeführt.

Beispiele und besonders bevorzugte Spezies der eingesetzten Säure sind die für Verfahren 1 angegebenen Beispiele und besonders bevorzugte Spezies.

Bei dem erfindungsgemäßen Verfahren 2 wird Säure vorzugsweise in Mengen von 0,05 bis 5,0 Gew.-%, besonders bevorzugt 0,5 bis 2,0 Gew.-%, bezogen auf das Gesamtgewicht an Saccharid (1), eingesetzt.

Die 2. Stufe des erfindungsgemäßen Verfahrens 2 wird bei einer Temperatur von vorzugsweise 85 bis 120°C, besonders bevorzugt 95 bis 110°C, und einem Druck von vorzugsweise 20 bis 200 hPa, besonders bevorzugt von 50 bis 150 hPa, durchgeführt.

Nach Beendigung der Umsetzung der 2. Stufe wird vorteilhafterweise die Säure neutralisiert und die erfindunsggemäße Organosiliciumverbindung isoliert.

Zur Neutralisation der Säure können alle in Verfahren 1 beschriebenen Basen eingesetzt werden.

Bei den im Verfahren 1 wie im Verfahren 2 eingesetzten Komponenten kann es sich jeweils um eine Art einer derartigen Komponente wie auch um ein Gemisch aus mindestens zwei Arten einer derartigen Komponente handeln.

Die nach den erfindungsgemäßen Verfahren 1 und 2 hergestellten, Glycosidreste aufweisende Organosiliciumverbindungen stellen jeweils Gemische verschiedener isomerer Formen dar und können nebeneinander mit α- und β-glycosidischen Bindungen vorliegen.

Die erfindungsgemäßen, Glycosidreste aufweisenden Organosiliciumverbindungen haben den Vorteil, daß sie mit

anderen Organosiliciumverbindungen sehr gut verträglich sind und sich somit beispielsweise für den Einsatz in Organopolysiloxan enthaltenden Zusammensetzungen eignen. Des weiteren haben die erfindungsgemäßen Organosiliciumverbindungen den Vorteil, daß sie weitgehend biologisch abbaubar sind. Darüberhinaus haben die erfindungsgemäßen, Glycosidreste aufweisenden Organosiliciumverbindungen den Vorteil, daß sie schaumstabilisierend wie auch schaumbildend wirken und als Netzmittel eingesetzt werden können.

Die erfindungsgemäßen, Glycosidreste aufweisenden Organosiliciumverbindungen können für alle Zwecke eingesetzt werden, für die bisher Alkyl(poly)glycoside verwendet wurden. So können die erfindungsgemäßen, Glycosidreste aufweisenden Organosiliciumverbindungen insbesondere als Tenside eingesetzt werden. Dabei zeigt es sich als vorteilhaft, daß sie mit anderen Tensiden sehr gut verträglich sind. Aufgrund der milden Tensidwirkung der erfindungsgemäßen Substanzen eignen sie sich ausgezeichnet für kosmetische und pharmazeutische Anwendungen. Da die erfindungsgemäßen, Glycosidreste aufweisenden Organosiliciumverbindungen selbst bzw. deren wäßrige Lösungen stark schäumen, eignen sie sich sehr gut als Additive für Reinigungsmittel. Des weiteren können die erfindungsgemäßen Organosiliciumverbindungen als Emulgatoren für synthetische und natürliche Öle sowie Wachse eingesetzt werden.

In den nachstehend beschriebenen Beispielen beziehen sich alle Angaben von Teilen und Prozentsätzen, falls nicht anders angegeben, auf das Gewicht. Des weiteren beziehen sich alle Viskositätsangaben auf eine Temperatur von 25°C. Sofern nicht anders angegeben, wurden die nachstehenden Beispiele bei einem Druck der umgebenden Atmosphäre, also etwa 1000 hPa, und bei Raumtemperatur, also bei etwa 20°C, bzw. bei einer Temperatur, die sich beim Zusammengeben der Reaktanden bei Raumtemperatur ohne zusätzliche Heizung oder Kühlung einstellt, durchgeführt.

**Beispiel 1**

A) Herstellung des Alkenylglycosids I

153 g $HO-CH_2CH_2O-CH_2CH=CH_2$ (im folgenden "Allylglykol" genannt) werden mit 99 g Glucose Monohydrat vermischt und bei einem Druck von 60 hPa und einer Temperatur von 65 °C erst für eine Dauer von einer Stunde, dann bei einem Druck von 60 hPa und einer Temperatur von 100°C für eine Dauer von 15 Minuten gerührt, wobei gleichzeitig destilliert wird (Destillat: 9,5 g eines Wasser/Allylglycol-Gemisches). Anschließend werden 2,0 g einer Lösung aus 1,0 g p-Toluolsulfonsäure, 0,4 g Wasser und 0,6 g Allylglykol zugegeben und bei einem Druck von 90 hPa und einer Temperatur von 100°C für eine Dauer von 3 bis 4 Stunden gerührt bis die Mischung klar ist, wobei gleichzeitig destilliert wird (Destillat: 14 g eines Wasser/Allylglycol-Gemisches). Die klare Mischung wird nun mit 1,0 g einer 30 %igen wäßrigen NaOH-Lösung neutralisiert und dann bei einem Druck von 1 hPa und einer Temperatur von 110°C nochmals destilliert. Es wird ein bei 100°C zähflüssiges und bei Raumtemperatur festes, glasartiges Produkt erhalten. über $^1H$-NMR wird eine nahezu 100%ige Ausbeute und ein durchschnittlicher Glycosidierungsgrad von 1,5 bestimmt.

43,4 g des oben beschriebenen Alkenylglycosids I werden auf 80°C erwärmt, mit 0,14 g einer Lösung von 1 g $H_2PtCl_6 \cdot 6H_2O$ in 99 g Isopropanol (Platingehalt der Lösung: 38 ppm) versetzt und innerhalb von 15 Minuten 51,8 g Hydrogenpentamethyldisiloxan zugetropft, wobei die Temperatur auf 100°C ansteigt. Anschließend wird die Mischung für eine Dauer von einer Stunde bei 80°C gerührt und dann bei einem Druck von 1 hPa und 60°C destilliert. Es werden 60 g einer Verbindung der durchschnittlichen Formel

$$H(C_6H_{10}O_5)_{1,5}-O-CH_2CH_2O-CH_2-CH_2-CH_2-Si(CH_3)_2-O-Si(CH_3)_3$$

erhalten, welche bei 80°C zähflüssig und klar und bei Raumtemperatur fest ist.

**Beispiel 2**

56,0 g des in Beispiel 1 unter A) beschriebenen Alkenylglycosids I werden auf 80°C erwärmt, mit 0,33 g einer Lösung von 1 g $H_2PtCl_6 \cdot 6H_2O$ in 99 g Isopropanol (Platingehalt der Lösung: 38 ppm) versetzt und innerhalb von 45 Minuten 166,6 g eines mit Trimethylsilylgruppen terminierten Dimethyl/methylhydrogenpolysiloxans mit 60 Siloxyeinheiten und einem Gehalt von Si-gebundenem Wasserstoff von 0,12 % zugetropft, wobei die Temperatur auf 100°C ansteigt. Anschließend wird die Mischung für eine Dauer von einer Stunde bei 80°C gerührt und dann bei einem Druck von 1 hPa und 60°C destilliert. Es werden 215 g eines mit Trimethylsilylgruppen terminierten Diorganopolysiloxans, welches als Diorganosiloxygruppen außer Dimethylsiloxygruppen Einheiten der durchschnittlichen Formel

$$H(C_6H_{10}O_5)_{1,5}-O-CH_2CH_2O-CH_2-CH_2-CH_2-SiCH_3=$$

aufweist, erhalten. Das erhaltene Organopolysiloxan ist bei 80°C zähflüssig und bei Raumtemperatur fest.

**Beispiel 3**

B) Herstellung eines hydroxyalkylfunktionellen Disiloxans

153 g Allylglykol und 0,72 g einer Lösung von 1 g $H_2PtCl_6 \cdot 6H_2O$ in 99 g Isopropanol (Platingehalt der Lösung: 38 ppm) werden unter Rühren auf 80°C erhitzt und während einer Dauer von 1 bis 1,5 Stunden 329,6 g Hydrogenpentamethyldisiloxan zugetropft, wobei die Temperatur auf 110°C ansteigt. Anschließend wird die Mischung für eine Dauer von einer Stunde bei 80°C gerührt und dann bei einem Druck von 1 hPa und 100°C destilliert. Es wird ein fast farbloses, klares Öl mit einer Viskosität von 9 $mm^2/s$ erhalten.

150 g des oben beschriebenen hydroxyalkylfunktionellen Disiloxans werden mit 39,6 g Glucose Monohydrat vermischt und bei einem Druck von 65 hPa und einer Temperatur von 60 °C erst für eine Dauer von einer Stunde, dann bei einem Druck von 65 hPa und einer Temperatur von 100°C für eine Dauer von 15 Minuten gerührt, wobei gleichzeitig destilliert wird (Destillat: 2,5 g Wasser). Anschließend werden 1,5 g einer Lösung aus 0,75 g p-Toluolsulfonsäure, 0,3 g Wasser und 0,45 g des oben beschriebenen hydroxyalkylfunktionellen Disiloxans zugegeben und bei einem Druck von 90 hPa und einer Temperatur von 100°C für eine Dauer von 5 Stunden gerührt (Destillat: 37 g Wasser). Die so erhaltene Mischung wird filtriert. Es werden 57 g eines Feststoffs erhalten, der zu 50 % aus einer Verbindung der durchschnittlichen Formel

$$H(C_6H_{10}O_5)_{1,5}\text{-}O\text{-}CH_2CH_2O\text{-}CH_2\text{-}CH_2\text{-}CH_2\text{-}Si(CH_3)_2\text{-}O\text{-}Si(CH_3)_3$$

und zu 50 % aus reinem Polysaccharid besteht.

**Patentansprüche**

1. Glycosidreste aufweisende Organosiliciumverbindungen, dadurch gekennzeichnet, daß es sich um solche der Formel

$$R^1{}_xR_{3-x}SiO\text{-}[(SiRR^1O)_m\text{-}(SiR_2O)_n]_y\text{-}SiR_{3-x}R^1{}_x \quad \text{(III)},$$

handelt, worin

R gleich oder verschieden sein kann und Wasserstoffatom oder gegebenenfalls substituierte Kohlenwasserstoffreste mit 1 bis 18 Kohlenstoffatomen bedeutet,
$R^1$ gleich oder verschieden sein kann und einen Rest der Formel

$$Z\text{-}(R^2O)_c\text{-}R^3\text{-} \quad \text{(II)}$$

bedeutet, worin
Z einen Glycosidrest bedeutet, der aus 1 bis 10 Monosaccharideinheiten aufgebaut ist,
$R^2$ gleich oder verschieden sein kann und Alkylenrest bedeutet,
c 0 oder eine Zahl von 1 bis 20 ist und
$R^3$ Alkylenrest bedeutet,
m gleich oder verschieden sein kann und 0 oder eine Zahl von 1 bis 200 ist,
n gleich oder verschieden sein kann und 0 oder eine Zahl von 1 bis 1000 ist,
x 0 oder 1 ist und
y 0 oder eine Zahl von 1 bis 1200 ist,

mit der Maßgabe, daß die Verbindung der Formel (III) mindestens einen Rest $R^1$ aufweist.

2. Glycosidreste aufweisende Organosiliciumverbindungen gemäß Anspruch 1, dadurch gekennzeichnet, daß, falls in Formel (III) m durchschnittlich verschieden von 0 ist, x 0 bedeutet.

3. Glycosidreste aufweisende Organosiliciumverbindungen gemäß Anspruch 1, dadurch gekennzeichnet, daß, falls in Formel (III) x durchschnittlich verschieden von 0 ist, m 0 bedeutet.

4. Verfahren zur Herstellung von Glycosidreste aufweisenden Organosiliciumverbindungen gemäß einem oder mehreren der Ansprüche 1 bis 3, dadurch gekennzeichnet, daß in einer

1. Stufe

Mono- und/oder Oligosaccharid (1) mit Verbindung (2) der Formel

$$HO\text{-}(R^2O)_c\text{-}R^4 \qquad (IV),$$

worin

$R^2$ gleich oder verschieden sein kann und Alkylenrest bedeutet,
c 0 oder eine Zahl von 1 bis 20 ist und
$R^4$ einen Alkenylrest bedeutet,
umgesetzt wird und in einer

2. Stufe
das in der 1. Stufe erhaltene Glycosid mit Si-gebundenen Wasserstoff aufweisender Organosiliciumverbindung (3) aus Einheiten der Formel

$$R^5_dH_eSiO_{\frac{4-d-e}{2}} \qquad (V)$$

umgesetzt wird, worin

$R^5$ gleich oder verschieden sein kann und gegebenenfalls substituierte Kohlenwasserstoffreste mit 1 bis 18 Kohlenstoffatomen bedeutet,
d 0, 1, 2 oder 3 ist und
e 0, 1, 2 oder 3 ist,

mit der Maßgabe, daß die Summe aus d und e kleiner oder gleich 3 ist und Organosiliciumverbindung aus Einheiten der Formel (V) pro Molekül mindestens ein Si-gebundenes Wasserstoffatom enthält.

**5.** Verfahren nach Anspruch 4, dadurch gekennzeichnet, daß als Mono- und/oder Oligosaccharid Glucose bzw. Glucose Monohydrat eingesetzt wird.

**6.** Verfahren zur Herstellung von Glycosidreste aufweisenden Organosiliciumverbindungen gemäß einem oder mehreren der Ansprüche 1 bis 3, dadurch gekennzeichnet, daß in einer

1. Stufe
Verbindung (2) mit Organosiliciumverbindung (3) umgesetzt wird und dann in einer
2. Stufe
die in der 1. Stufe erhaltene Organosiliciumverbindung mit Saccharid (1) umgesetzt wird.

**7.** Verwendung der Glycosidreste aufweisenden Organosiliciumverbindungen gemäß einem oder mehreren der Ansprüche 1 bis 3 oder hergestellt gemäß Anspruch 4 oder 6 als Tenside.

## Claims

**1.** Organosilicon compounds containing glycoside radicals, characterized in that they are those of the formula

$$R^1{}_xR_{3-x}SiO\text{-}[(SiRR^1O)_m\text{-}(SiR^2O)_n]_y\text{-}SiR_{3-x}R^1{}_x \qquad (III)$$

in which

R can be identical or different and denotes a hydrogen atom or optionally substituted hydrocarbon radicals having 1 to 18 carbon atoms,
$R^1$ can be identical or different and denotes a radical of the formula

$$Z\text{-}(R^2O)_c\text{-}R^3\text{-} \qquad (II)$$

in which

Z denotes a glycoside radical which is built up from 1 to 10 monosaccharide units,

$R^2$ can be identical or different and denotes an alkylene radical,
c is 0 or a number from 1 to 20 and
$R^3$ denotes an alkylene radical,
m can be identical or different and is 0 or a number from 1 to 200,
n can be identical or different and is 0 or a number from 1 to 1000,
x is 0 or 1 and
y is 0 or a number from 1 to 1200,

with the proviso that the compound of the formula (III) contains at least one radical $R^1$.

**2.** Organosilicon compounds containing glycoside radicals according to Claim 1, characterized in that, if 0 m in formula (III) is on average other than 0, x denotes 0.

**3.** Organosilicon compounds containing glycoside radicals according to Claim 1, characterized in that, if x in formula (III) is on average other than 0, m denotes 0.

**4.** Process for the preparation of organosilicon compounds containing glycoside radicals according to one or more of Claims 1 to 3, characterized in that, in a 1st stage
a mono- and/or oligosaccharide (1) is reacted with a compound (2) of the formula

$$HO\text{-}(R^2O)_c\text{-}R^4 \qquad (IV)$$

in which

$R^2$ can be identical or different and denotes an alkylene radical,
c is 0 or a number from 1 to 20 and
$R^4$ denotes an alkenyl radical,

and in a
2nd stage
the glycoside obtained in the 1st stage is reacted with an organosilicon compound (3) containing Si-bonded hydrogen, comprising units of the formula

$$R^5_dH_eSiO_{\frac{4-d-e}{2}} \qquad (V)$$

in which

$R^5$ can be identical or different and denotes optionally substituted hydrocarbon radicals having 1 to 18 carbon atoms,
d is 0, 1, 2 or 3 and
e is 0, 1, 2 or 3,

with the proviso that the sum of d and e is less than or equal to 3 and the organosilicon compound comprising units of the formula (V) contains at least one Si-bonded hydrogen atom per molecule.

**5.** Process according to Claim 4, characterized in that glucose or glucose monohydrate is employed as the mono- and/or oligosaccharide.

**6.** Process for the preparation of organosilicon compounds containing glycoside radicals according to one or more of Claims 1 to 3, characterized in that, in a
1st stage
a compound (2) is reacted with an organosilicon compound (3), and then in a
2nd stage
the organosilicon compound obtained in the 1st stage is reacted with a saccharide (1).

**7.** Use of the organosilicon compounds containing glycoside radicals according to one or more of Claims 1 to 3 or prepared according to Claim 4 or 6 as surfactants.

## Revendications

1. Composés organosiliciés comportant un reste glycosidique, caractérisés en ce qu'il s'agit de ceux de formule

$$R^1{}_xR_{3-x}\text{-SiO-}[(SiRR^1O)_m\text{-}(SiR^2O)_n]_y\text{-}SiR_{3-x}R^1{}_x \qquad (III),$$

où

R peut être identique ou différent et représente un atome d'hydrogène ou un radical hydrocarbure facultativement substitué avec 1 à 18 atomes de carbone,
$R^1$ peut être identique ou différent et représente un radical de formule

$$Z\text{-}(R^2O)_c\text{-}R^3\text{-} \qquad (II)$$

où
Z représente un reste glycosidique qui est construit à partir de 1 à 10 unités monosaccharides,
$R^2$ peut être identique ou différent et représente un radical alcoylène,
c est 0 ou un nombre de 1 à 20, et
$R^3$ représente un radical alcoylène,
m peut être identique ou différent et est 0 ou un nombre de 1 à 200,
n peut être identique ou différent et est 0 ou un nombre de 1 à 1000,
x est 0 ou 1, et
y est 0 ou nombre de 1 à 1200,
avec la condition que le composé de formule (III) comporte au moins un radical $R^1$.

2. Composés organosiliciés comportant un reste glycosidique suivant la revendication 1, caractérisés en ce que, si dans la formule (III), m est en moyenne différent de 0, x représente 0.

3. Composés organosiliciés comportant un reste glycosidique suivant la revendication 1, caractérisés en ce que, si dans la formule (III), x est en moyenne différent de 0, m représente 0.

4. Procédé de préparation de composés organosiliciés comportant un reste glycosidique suivant l'une ou plusieurs des revendications 1 à 3, caractérisé en ce que, dans une première étape
on fait réagir un mono- et/ou oligosaccharide (1) avec un composé (2) de formule

$$HO\text{-}(R^2O)_c\text{-}R^4 \qquad (IV),$$

où

$R^2$ peut être identique ou différent et représente un radical alcoylène,
c est 0 ou un nombre de 1 à 20, et
$R^4$ représente un radical alcoylène, et dans une deuxième étape

le glycoside obtenu à la première étape est mis à réagir avec un composé organosilicié (3) comportant des hydrogènes à liaison Si, constitué d'unités de formule

$$R^5{}_dH_eSiO_{4-d-e} \qquad (V)$$

où

$R^5$ peut être identique ou différent et représente un radical hydrocarbure facultativement substitué avec 1 à 18 atomes de carbone,
d est 0, 1, 2 ou 3, et
e est 0, 1, 2 ou 3,

avec la condition que la somme de d et e soit égale ou inférieure à 3 et que le composé organosilicié constitué d'unités de formule (V) contienne, par molécule, au moins un atome d'hydrogène à liaison Si.

5. Procédé suivant la revendication 4, caractérisé en ce que l'on utilise du glucose et respectivement, du glucose monohydraté comme mono- et/ou oligosaccharide.

**6.** Procédé de préparation de composés organosiliciés comportant un reste glycosidique suivant l'une ou plusieurs des revendications 1 à 3, caractérisé en ce que,
dans une première étape
on fait réagir le composé (2) avec le composé organosilicié (3) et ensuite,
dans une deuxième étape
le composé organosilicié obtenu à la première étape est mis à réagir avec le saccharide (1).

**7.** Utilisation des composés organosiliciés comportant un reste glycosidique suivant l'une ou plusieurs des revendications 1 à 3 ou préparés suivant la revendication 4 ou 6, comme tensioactif.